# EUROPEAN PATENT SPECIFICATION

(11) **EP 4 023 076 B1**
(45) Date of publication and mention of the grant of the patent: **04.03.2026**
(21) Application number: 19943225.3
(22) Date of filing: 30.08.2019
(51) Int. Cl.: A23L 29/238, A23L 29/256, A23G 3/48, A23G 4/08, A61K 9/34, A61K 9/68, A23L 21/10, A23L 21/18, A23G 3/34, A23G 3/36, A23G 3/40, A23G 3/42, A23G 3/54, A23L 29/206, A61J 3/06

(54) **CHEWABLE HEAT-RESISTANT ORAL DOSAGE FORM WITH AN AGAR MATRIX AND PRODUCTION METHOD FOR OBTAINING THE CHEWABLE ORAL DOSAGE FORM**
KAUBARE HITZEBESTÄNDIGE ORALE DARREICHUNGSFORM MIT EINER AGAR-MATRIX UND HERSTELLUNGSVERFAHREN ZUR HERSTELLUNG DER KAUBAREN ORALEN DARREICHUNGSFORM
FORME ORALE MASTICABLE, THERMORÉSISTANTE, AVEC UNE MATRICE D'AGAR ET PROCÉDÉ DE FABRICATION POUR OBTENIR LADITE FORME ORALE MASTICABLE

(43) Date of publication of application: 06.07.2022
(73) Proprietor: Procaps S.A., Barranquilla, 080013 (CO)
(72) Inventor: OCAMPO SALGADO, Andrea, Bogotá, 110111 (CO); DOMINGUEZ MARIN, Lady Elizabeth, Bogotá, 110111 (CO); BERNAL GARCÍA, Gustavo Adolfo, Bogotá, 110111 (CO); CANO CABRERA, Juan Carlos, Barranquilla (CO)
(74) Representative: Schüssler, Andrea
(86) International application number: PCT/IB2019/057350
(87) International publication number: WO 2021/038280

(56) References cited:
- EP-A1- 1 669 090
- EP-A1- 2 201 956
- EP-A2- 1 074 183
- EP-A2- 1 074 183
- WO-A1-98/34499
- CA-A1- 2 999 313
- US-A1- 2002 168 460
- SOUSA ANA M M ET AL.: "The influence of locust bean gum on native and alkali-modified agar gels", FOOD HYDROCOLLOIDS, vol. 44, 1 February 2015 (2015-02-01), pages 461 - 470, XP055794571, ISSN: 0268-005X, DOI: 10.1016/j.foodhyd.2014.10.020

## Description

### FIELD OF THE INVENTION

The object of the present invention is a chewable oral form and a process for shaping a chewable oral form such as a gum, with characteristics similar to those of gelatin and with a pleasant texture and taste.

In particular, the invention comprises a process of shaping an oral form that comprises a first stage of hydration of the agar-agar and locust bean gum gelling agent; a second stage of incorporation of components of the agar matrix; a third stage of incorporation of the active ingredient; a fourth stage of cooking the mixture; a fifth stage of flavoring; a sixth stage of molding and gelling; and, a seventh stage of demoulding, degreasing and drying to provide a composition with an agar-agar matrix and locust bean gum, where the texture allows it to be chewed and ingested without difficulty.

Similarly, the oral form according to the present invention comprises an agar-agar matrix in combination with gums such as locust bean gum and/or sugar; glycerin; inulin; flavors; citric acid; coatings and water to complete the composition that includes active ingredients.

The shaped form incorporates a composition based on active ingredients of a nutraceutical or pharmaceutical nature avoiding crosslinking problems so that active ingredients with melting points higher than 40° C can be incorporated.

Similarly, this also includes a chewable oral form suitable for the intake of active ingredients in vegans or those who by culture do not consume substances of animal origin.

The chewable oral form is made from an agar matrix, where the texture has been modified in such a way that it can be chewed and ingested by any type of patient.

The agar matrix chewable oral form provides a strong rubbery texture upon modification based for example on packaging, shipping and ambient temperature.

It has adequate stability at a temperature of 40° C, important in tropical countries, so that the oral chewable form preserves its structure under these conditions and at the same time is an easy-to-ingest alternative for the general consumer, preserving a soft texture to the chew.

Additionally, the oral chewable form melts at average body temperature with excellent flavor release.

Thus, a short-textured chewable oral form is provided, forming a stable product that is easy to chew and ingestable.

The oral form comprises an active ingredient selected from vitamin D2, vitamin A, vitamin C, vitamin D and its derivatives and / or analogues, vitamin E, B complex vitamins, such as vitamins B1, B3, B5, B9 and B12 and vitamin K2; probiotics; minerals selected from zinc, calcium, magnesium, iron, selenium, chromium, phosphorus, potassium, copper; omega 3 fatty acids selected from EPA and DHA; folic acid, iodine, biotin, choline, lutein or their combinations.

The chewable oral form of agar matrix according to the present invention comprises a formulation that achieves a balance in the mixture of the aforementioned components to provide a matrix that can be industrially processed and that has stability during a shelf life of 2 years.

The present invention is thus applicable to the nutritional or health area since it provides an oral form with outstanding texture, visual shape and flavor capable of supplying the desired amount of an active ingredient.

### BACKGROUND OF THE INVENTION.

Adding nutrients to easily ingested products is a practice that dates back to the early 19th century, when the French chemist Boussingault recommended adding iodine to table salt to prevent goiter in South America.

In 1918, in Denmark, margarine was fortified with concentrated vitamin A and in 1931 in the USA, whole milk was fortified with vitamin D. As for cereals, the fortification of breakfast cereals began in the middle of the 20th century, specifically in 1941.

Another of the first products that were manufactured under the name of fortified foods was a mixture of basic flour with fish meals to achieve a higher protein intake, another food fortification process was to add iodine to milk to prevent problems of hypothyroidism in endemic areas.

Consumers are increasingly aware of the need to eat foods that provide functional elements to their diet that are beneficial for their health and well-being. Therefore, a current need is good nutrition, with enriched forms that help add the necessary nutritional requirements to the human body.

In Latin America, in general, child malnutrition is a recurring social problem that affects the majority of the population regardless of socio-economic levels. Poor nutrition is mainly caused by not consuming food with enough nutrients required by the body (proteins, vitamins, minerals and antioxidants, among others).

It was also found that in some Latin American countries such as Colombia, 32.2% of children between 1 and 4 years old and 37.6% of children between 5 and 12 years old suffer from anemia due to poor nutrition (Instituto Colombiano de Bienestar Familiar ICBF, 2005) and that children in general are deficient in vitamin by not consuming foods that provide them with a balanced diet

Several oral forms of ingestion have been known with different sensory characteristics, most of them rejected by the consumer for their appearance, taste and texture.

There are developments in the state of the art, such as that disclosed in European patent EP1074183 by Aoki, Miwa et al., which illustrates a food gelatin containing 0.3-1.2% by weight of agar, 0.01-0.6% by weight of xanthan gum and 0.01-0.6% by weight of locust bean gum as gelling agents, characterized by having sarcocarp like granules and texture obtained by a process in which gelling agents are mixed in a ratio of xanthan gum to locust bean gum between 1 to 4 and 4 to 1 in an amount of agar that exceeds a total amount of xanthan gum and locust bean gum, with the mixture heated to obtain a solution, after which this solution is cooled to obtain a gel, and the resulting gel is frozen and thawed to obtain a gum. This development is viable in the conformation and molding of gums but it is made based on gelatin of animal origin, which is not suitable for all types of consumers due to its origin, appearance, taste and texture.

US 2002/0168460 discloses a starch molded gelled dried fruit product, wherein the gelling system is based on pectin and carrageenan. It is mentioned that minor nutricitional ingredients such as vitamin can be added.

Another development of the art is defined in US patent US7067150 to Farber, Michael et al., Which discloses oral delivery systems for functional ingredients, such as drugs, nutritional supplements, botanicals and vitamins.

Delivery systems comprise an ingestible matrix within which the functional ingredients are dispersed substantially uniformly and completely and in which degradation of the functional ingredients is minimized where the matrix comprises one or more carbohydrates; one or more sugar, sugar syrup and/or sugar alcohol; one or more hydrocolloids comprising gelatin; one or more polyhydric alcohols; one or more sources of mono or divalent cations, and water.

The combination of carbohydrates and hydrocolloids in the matrix ensures that the delivery system easily retains the solvent component and therefore prevents separation of the solvent from other components of the matrix.

The invention also provides methods for preparing and using delivery systems. This development also bases the matrix of the oral form in gelatin of animal origin, so it is not a suitable solution for all types of consumer.

As shown by said prior art, within the oral forms with greater acceptance by the consumer, there are gums made from gelatin, due to their gelling properties, which are the preferred alternative in formulations of pharmaceutical or nutraceutical forms. Suitable gelatin substitutes with gelling properties are starch, carrageenan, and agar.

Agar is an effective gelling agent of vegetable origin, on which it is possible to produce a non-sticky gel with a crunchy texture. Agar is made from red algae including *Gelidium, Euchema,* and *Gracilaria* and has special properties, unlike other gelling agents, and is applicable to various uses.

Gums made from agar are positioned in a very dynamic market that is at the forefront of new consumer requirements, where consumer trends today are marked by having products that can be verifiable (Non-GMO, Organic, Halal , Kosher), so that a material from a plant has a greater facility to be verified compared to any product of animal origin. Other types of factors to evaluate include consumers based on religious groups, ethnic groups, vegetarians/vegans, health reasons associated with the needs of patients and/or consumers.

Additionally, the stability and performance of the agar, the low sensitivity to heat or humidity, its ability to avoid cross-linking, the low sensitivity to pH, and the advantages compared to the reduction of the migration of water from the outside as well as high mechanical strength, advantageous organoleptic properties such as neutral taste, odorlessness, clarity/gloss and easy chewing and ingestion among others, position it as an alternative of choice due to its physical and chemical properties that favor production processes and facilitate its acceptance by the consumer in general.

On the other hand, the need to supply vitamins, minerals, botanical-based nutraceutical compositions or simply pharmaceutical active ingredients that are easy to ingest, is of vital importance in recent times, in a time where stress, fatigue, difficulty sleeping which are one of the biggest population problems, where minerals and vitamins are a natural alternative to meet this need.

Additionally, several clinical and meta-analysis studies have been carried out that show a significant impact on the reduction of migraines, lower probability of colon rectal cancer, improvement of calcium absorption in the bones and constipation. Due to changes in eating habits and the decrease in the magnesium content in food, the daily intake of magnesium in the population is deficient, for which it is beneficial to supplement.

The chewable forms are a solution for the administration of vitamins, minerals, nutraceutical compositions based on botanicals or simply active ingredients of an easy-to-take pharmaceutical nature.

Chewable forms such as gums are a comfortable, available and convenient alternative as a food supplement, as they can be consumed at any time, no water is required and they are easy to swallow. However, for active ingredients with melting points above 40 ° C, the chewable gel forms on the market are not a viable alternative.

Therefore, another important problem to be solved is that in addition to forming a chewable oral form based on gel of plant origin, it is to create a chewable oral gel form of plant origin that also includes a matrix that overcomes the cross-linking problems very common in gelatin-based preparations and that can integrally incorporate active ingredients with melting points higher than 40°C, where the chewable gel forms on the market offer no alternative.

### OBJECTS OF THE INVENTION.

Accordingly, a first object of the present invention is to avoid the disadvantages of the prior art.

More particularly, a main object of the present invention is to generate a chewable oral form with a matrix of plant origin such as agar and to formulate within said matrix natural or synthetic compounds with nutritional or pharmaceutical properties, whose organoleptic properties provide a better experience to the consumer.

In particular, an oral form with a matrix of plant origin with similar characteristics compared to gelatin of animal origin, which overcomes the very common cross-linking problems in gelatin preparations, so that it can incorporate active ingredients with melting points above 40°C.

A no less important object is to form a chewable oral form suitable for the intake of active ingredients in vegans or those who by culture do not consume substances of animal origin where the texture has been modified in such a way that it is susceptible to be chewed and ingested in any type of patient.

Another important object comprises an agar matrix chewable oral form that provides a strong rubbery texture upon modification based for example on packaging, transportation, ambient temperature and stable, important in tropical countries, so that the chewable oral form retain its shape and at the same time for consumption, retain a soft texture when chewing and melting at body temperature with excellent flavor release.

Particularly an object of the invention is a process for shaping a chewable oral form that comprises a first stage of hydration of the agar-agar and locust bean gum gelling agent; a second stage of incorporation of components of the agar matrix; a third stage of incorporation of the active ingredients; a fourth stage of cooking the mixture; a fifth stage of flavoring; a sixth stage of molding and gelling; and, a seventh stage of demoulding, degreasing and drying.

An also important object is to form a chewable oral form comprising an agar-agar matrix in combination with gums such as locust bean gum and/or sugar; glycerin; inulin; flavors; citric acid; coatings and water to complete the composition that includes an active ingredient selected from vitamin D2, vitamin A, vitamin C, vitamin D and its derivatives and / or analogues, vitamin E, B complex vitamins, such as vitamins B1, B3, B5, B9 and B12 and vitamin K2; probiotics; minerals selected from zinc, calcium, magnesium, iron, selenium, chromium, phosphorus, potassium, copper; omega 3 fatty acids selected from EPA and DHA; folic acid, iodine, biotin, choline, lutein or their combinations.

The novel features that are considered as the foundation of the invention are set forth in particular in the appended claims and the additional advantages thereof, will be better understood on the following detailed description with the preferred embodiments.

### DETAILED DESCRIPTION OF THE INVENTION

The object of the present invention is a chewable oral form with an agar-agar matrix in combination with gums such as locust bean gum and/or sugar; glycerin; inulin; flavors; citric acid; coatings and water to complete the composition that includes an active ingredient selected from vitamin D2, vitamin A, vitamin C, vitamin D and its derivatives and / or analogues, vitamin E, B complex vitamins, such as vitamins B1, B3, B5, B9 and B12 and vitamin K2; probiotics; minerals selected from zinc, calcium, magnesium, iron, selenium, chromium, phosphorus, potassium, copper; omega 3 fatty acids selected from EPA and DHA; folic acid, iodine, biotin, choline, lutein or their combinations.

In particular, the invention comprises an oral form with a matrix of plant origin, so as to provide a composition based on active ingredients of a nutraceutical or pharmaceutical nature so that it can incorporate active ingredients with melting points above 40°.

The agar matrix chewable oral form provides a strong rubbery texture to modification based for example on packaging, transportation, ambient temperature and stable at a temperature of 40° C, important in tropical countries, so that the oral chewable form retain its shape and at the same time for consumption, retain a smooth texture when chewing, melting at body temperature with excellent flavor release.

Thus, a short-textured chewable oral form is formed, forming a product that is easy to chew and swallow. Agar is a non-branched polysaccharide obtained from the cell wall of several species of algae of the *Gelidium, Euchema* and *Gracilaria* genera, resulting, depending on the species, with characteristic properties and colors.

Chemically agar is a polymer of galactose subunits; a result of the mixture of two types of polysaccharides: agaropectin and agarose. The polysaccharides in agar are part of the algae cell wall structure. In the presence of water it has gelatinous properties and is widely used in bacteriology as a culture medium.

However, the present invention aims to use agar for the development of a matrix that will provide gelatinous characteristics to a composition that can include nutraceutical or pharmaceutical active ingredients. Gums, for their part, are resinous substances, polysaccharides soluble in water, with sticky characteristics, have high molecular weight and are structurally complex. Its consistency is solid, but it can vary according to its origin, the process conditions and particularly it is characterized by elasticity, they have the property of forming gels and increasing viscosity, hence they are widely used in the food and pharmaceutical industry, due to to their emulsifying, stabilizing and thickening properties. Some examples of gums for food and pharmaceutical use include but are not limited to: gums extracted from marine plants (such as agar and alginates), gums extracted from seeds of land plants, gums obtained as exudates from terrestrial plants (such as gum arabic, tragacanth gum, carob gum), gums obtained from microbiological processes, such as xanthan gum, among other types of gums available and applicable in both industries within these classifications The carob gum stands out for the present invention, which behaves as a coadjuvant agent which does not add to the gel strength in a mixture with agar but it does allow to modify the structure of the agar allowing it to resemble gelatin in the process and generating a less short structure at the time of the bite.

For the present invention, the manufacture of the oral chewable form is developed from the combination of a gelling agent such as agar-agar and a gum such as locust bean gum. The carob tree gum is solubilized in water from 80° C, while the agar at 75° C, the melting point of both occurs at 92° C (at 2,600 m a.s.l.) and its gelling begins at 75° C. A combination of agar-agar and locust bean gum is provided under the trademark AGARO ID 300^{®}.

As the solids increase in the mixture containing these gelling agents, the gelling temperature will increase as well. The importance of the point at which gelling begins is that if the process involves movement of the mixture, the gel that began to form at high temperatures will shear and the final product will present a softer texture, with less gel strength and with a high probability of syneresis.

Various types of natural or artificial sweeteners are used for the oral chewable form. Such natural sweeteners comprise sugars based on cane sugar, tapioca syrup and inulin, as well as sweeteners such as stevia or the like as long as it manages to stabilize the matrix of the agar-based gelling agent.

Sugar-based sweeteners such as cane sugar, tapioca syrup, and inulin, allow for a firm gel structure with optimal handling and sensory characteristics. The balance between the components of the matrix and their interaction with the active ingredients is essential to obtain a stable gel matrix. Other types of sweeteners are suitable for people with restrictions, such as diabetics.

Among the active ingredients that are suitable to form the oral chewable form of the present invention, vitamins such as vitamin D2, vitamin A, vitamin C, vitamin D and its derivatives and/or analogues, vitamin E, vitamins of the B complex, such as vitamins B1, B3, B5, B9 and B12 and vitamin K2, probiotics, minerals selected from zinc, calcium, magnesium, iron, selenium, chromium, phosphorus, potassium, copper; omega 3 fatty acids selected from EPA and DHA, folic acid, iodine, biotin, choline and lutein.

The process of shaping the oral chewable form then comprises: (i) A first stage of hydration of the agar-agar and locust bean gum gelling agent, which includes mixing agar-agar in combination with locust bean gum (AGAROID 300^{®}) between 0.1% and 4.0%, particularly between 0.2% and 2.0% and more particularly between 0.3% and 1.8% of the total weight of the composition, with an amount of sweeteners in a range of between 6% and 10% until a homogeneous mixture is obtained, and then add a quantity of water and stir until complete dissolution of the mixture;
(ii) A second stage of incorporation of components of the agar matrix, where to the mixture of the first stage (i), glycerin is added in an amount between 1% and 3% of the total composition in combination with liquid inulin in an amount between 7% and 12% of the total composition and inulin powder in an amount of between 5% and 10% of the total composition and optionally mix with an amount of sweetener between 20% and 28% of the total composition, and then stir until complete dissolution;
(iii) A third stage of incorporation of the active ingredient, where previously an amount of the active ingredient and optionally an amount of sweetener of between 7% and 10% of the total composition are mixed until a homogeneous mixture is obtained and subsequently added to the mixture of the second stage (ii) and stirred until its complete incorporation;
(iv) A fourth stage of cooking the mixture obtained in the third stage (iii) at a temperature between 80° C and 100° C for 30 ± 5 minutes, where the solids must comprise between 72° and 74° Brix; and
(v) A fifth stage of flavoring that includes transferring the cooked paste from the fourth stage (iv) to a flavoring tank and adding black carrot in an amount of between 0.1% and 1%, particularly between 0.2% and 0.4 % in combination with flavoring in an amount between 0.1% and 1%,
particularly between 0.2% and 0.4 % in combination with flavoring in an amount between 0.1% and 1%, particularly between 0.4% to 0.8% and citric acid in an amount between 0.23% and 0.29%. the addition of citric acid allows a flavor balance to be conferred on the product, where the acid is finally added in the fifth stage and after all the other added components are fully incorporated into the mixture.

Once the mixture is prepared, the shaping of the final oral form should begin. Said final oral form may comprise shapes and colors based on the type of marketing or destination. This requires a highly stable wax-based coating to provide gloss, moisture resistance and excellent lubrication to control piece-to-piece adhesion. Said coating may comprise a coating based on a blend of natural oils and waxes and/or sweeteners, mannitol, or powdered flavors and may be formulated with additional combined flavors.

Thus, the process then additionally comprises the following steps:
(vi) A sixth stage of molding and gelling where the mixture of the fifth stage is deposited in molds at a temperature of between 75° and 89° C and its temperature for gelling is reduced to a temperature of between 2° C to 8° C; and,
(vii) A seventh stage of demoulding, degreasing and drying, which includes demoulding followed by degreasing, and then drying by spreading the chewable oral forms in drying racks or baskets at 30 ± 3° C temperature and 25 ± 5% relative humidity.

In the sixth stage, an additional function to the flavor of citric acid is to increase the solubility of the active ingredient added in the third stage. For the gelling of the sixth stage, the cooling of the molds comprises arranging various means such as cold rooms, air tunnels and the like, where the molds are induced to lower their temperature gently and slowly. For the degreasing of the seventh stage, it is arranged in a rotating drum where the product in baskets, rotate to homogenize the fat on the surface of the chewable forms.

For the seventh stage drying, a drying tunnel can be provided under special conditions of relative humidity 25 ± 5° C, with a temperature of between 30 ± 3° C and air velocity in laminar flow of 0.15 m³/sec.

Optionally the oral chewable form can also be sweetened. For this purpose, the process also includes:
(viii) An eighth stage of sweetening and packaging, where the dry chewable oral forms are introduced to the sweetening equipment and the sweetener is added until the coating is complete and for this the steam is adjusted between 275 Pa to 413 Pa, with a speed of 1 to 5 Kg unit/min and an amount of sweetener between 6% and 10%, where once sweetened, they are inspected and packaged.

Based on the process described above, a chewable oral form is obtained that comprises an agar-agar matrix in combination with locust bean gum and/or sugar; which includes glycerin; liquid inulin and powder inulin; an active ingredient, flavors; citric acid; coating and water to complete the composition.

The critical points for obtaining the chewable oral form according to the present invention with firmness characteristics, is that they do not present syneresis over time and in turn have a good behavior throughout the process are the following:
- Correct hydration of the gelling agent.
- Fulfill the established cooking time and temperature.
- Maintain the recommended temperature in the system avoiding gelling and subsequent shearing due to the gel process.
- The drying process must be gentle and at the same time efficient so that excess water is removed up to the established level.

Following the procedure with attention to the recommendations in each step, you will obtain a firm and stable product over time. Thus, a chewable oral form has been formed to provide a composition made from magnesium with an agar matrix, where the texture is capable of being chewed and ingested effectively.

One of the parameters showing the properties of the agar matrix chewable dosage form is the strength of the gel which indicates the solidness. In general, gel strength is proportional to the concentration of agar in a solution. Therefore, it was experimentally proven that if agar with high gel strength is used, a chewable form with the desired solidity can be obtained from a small amount of agar.

The oral chewable form comprises a composition of a range of 0.1% and 4.0% of agar-agar gelling agent in combination with locust bean gum; sweetener in a range of between 6% to 30%; glycerin in a range of between 1% and 3%; liquid inulin in a range of between 7% and 12%; inulin powder in a range of between 5% and 10%; flavoring in a range of between 0.1% and 1%; citric acid in a range of 0.1% and 0.5%; wax-based coating in a range of between 0.5% and 3%; black carrot in a range of 0.1% and 1%; in combination with active ingredients between 10% and 60% and more preferably between 25% and 35%; and water to complete the composition.

Among others, the flavors of the chewable form of the present invention can include: strawberry, watermelon, mango, lemon, peppermint, orange, passion fruit, banana, coconut, acerola, blueberry, currant, mandarin, papaya, grapefruit, acai, chamomile and ginger.

In the same way, the fruit extracts may comprise various extracts of sweet fruits, citrus, berries, combination of fruits and all fruits with suitable flavor characteristics as well as botanical extracts.

The active ingredients comprise vitamins selected from vitamin D2, vitamin A, vitamin C, vitamin D and its derivatives and / or analogues, vitamin E, B-complex vitamins, such as vitamins B1, B3, B5, B9 and B12 and vitamin K2, probiotics, minerals selected from zinc, calcium, magnesium, iron, selenium, chromium, phosphorus, potassium, copper; omega 3 fatty acids such as EPA and DHA, folic acid, iodine, biotin, choline, and lutein.

### EXAMPLE 1

Examples 1-2 and 7-14 do not form part of the present invention. A chewable oral form according to the present invention in an example illustration, comprises a range of 0.1% and 4.0% of agar-agar gelling agent in combination with locust bean gum; sweetener in a range of between 6% to 30%; glycerin in a range of between 1% and 3%; liquid inulin in a range of between 7% and 12%; inulin powder in a range of between 5% and 10%; flavoring in a range of between 0.1% and 1%; citric acid in a range of 0.1% and 0.5%; wax-based coating in one range between 0.5% and 3%; black carrot in a range of 0.1% and 1%; in combination with active ingredients from table 1, and water until completing the composition.

**Table 1**

| | | |
|---|---|---|
| CHEWABLE AGAR MATRIX | Vitamin D2 | Ergocalciferol |
| | Probiotic | *Bacillus coagulans* |
| | Extract of fruits and botanicals | Mixture of fruits and botanicals |

### EXAMPLE 2

The composition of the oral chewable form of Example 1 comprising active ingredients from Table 2.

**Table 2**

| | | |
|---|---|---|
| CHEWABLE AGAR MATRIX | Probiotic | *Bacillus coagulans* |
| | Extract of fruits and botanicals | Mixture of fruits and botanicals |

### EXAMPLE 3

Another illustration of the composition of the oral chewable form of Example 1 comprising the active ingredients of Table 3.

**Table 3**

| | | |
|---|---|---|
| CHEWABLE AGAR MATRIX | Vitamin C | Ascorbic acid |
| | Zinc | Zinc citrate |

### EXAMPLE 4

The composition of the oral chewable form of Example 1 comprising active ingredients based on fatty acids from Table 4.

**Table 4**

| | | |
|---|---|---|
| CHEWABLE AGAR MATRIX | Omega 3 | Fish Oil |
| | DHA | Fish Oil |
| | EPA | Fish Oil |

### EXAMPLE 5

The composition of the oral chewable form comprising active ingredients based on magnesium and vitamins from Table 5.

**Table 5**

| CHEWABLE AGAR MATRIX | Calcium | Calcium Phosphate |
|---|---|---|
| | Magnesium | Magnesium Citrate |
| | Vitamin D | Cholecalciferol |

### EXAMPLE 6

The composition of the oral chewable form of Example 1 comprising active ingredients based on Table 6.

**Table 6**

| | | |
|---|---|---|
| CHEWABLE AGAR MATRIX | Vitamin A | Vitamin A Palmitate |
| | Vitamin C | Ascorbic Acid |
| | Vitamin D | Cholecalciferol |
| | Vitamin E | d-α-Tocopheryl acetate |
| | Folic Acid | Folic Acid |
| | Vitamin B12 | Cyanocobalamin |
| | Vitamin B5 | Calcium D- Pantothenate |
| | Calcium | Tricálcium Phosphate |
| | Iodine | Potassium Iodide |
| | Zinc | Zinc Citrate |

### EXAMPLE 7

Another illustration of the composition of the oral chewable form of Example 1 comprising active ingredients from Table 7.

**Table 7**

| | | |
|---|---|---|
| CHEWABLE AGAR MATRIX | Vitamin A | Beta-carotene |
| | Vitamin C | Ascorbic acid |
| | Vitamin D2 | Ergocalciferol |
| | Vitamin E | d-α-Tocopheryl acetate |
| | Vitamin K2 | Menaquinone |
| | Vitamin B1 | Thiamine Mononitrate |
| | Vitamin B3 | Niacinamide |
| | Vitamin B6 | Pyridoxine HCI |
| | Vitamin B9 | Calcium Folate |
| | Vitamin B12 | Cyanocobalamin |
| | Biotin | Biotin |
| | Vitamin B5 | Pantothenic Acid |
| | Choline | Choline Tartrate |
| | Iodine | Potassium Iodide |
| | Magnesium | Magnesium Oxice |
| | Selenium | L- selenomethionine |
| | Zinc | Zinc Citrate |
| | Probiotic | *Bacillus coagulans* |
| | Ginger | Ginger juice |
| | Fruits and botanicals | Mixture of fruits and botánicals |

### EXAMPLE 8

The composition of the oral chewable form of example 1 comprising a combination of active ingredients from Table 8.

**Table 8**

| | | |
|---|---|---|
| CHEWABLE AGAR MATRIX | Vitamin A | Beta-carotene |
| | Vitamin C | Ascórbic ácid |
| | Vitamin D2 | Ergocalciferol |
| | Vitamin E | d-α-Tocopheryl acetate |
| | Vitamin K2 | Menaquinone |
| | Vitamin B1 | Thiamine Mononitrate |
| | Vitamin B3 | Niacinamide |
| | Vitamin B6 | Pyridoxine HCI |
| | Vitamin B9 | Calcium Folate |
| | Vitamin B12 | Cyanocobalamin |
| | Biotin | Biotin |
| | Vitamin B5 | Pantothénic Acid |
| | Choline | Choline Tartrate |
| | Iodine | Potassium Iodide |
| | Magnesium | Magnesium Oxice |
| | Selenium | L- selenomethionine |
| | Zinc | Zinc Citrate |
| | Probiotic | *Bacillus coagulans* |
| | Lutein | Lutein |
| | Blueberries | Organic Blueberries |
| | Fruits and botanicals | Mixture of fruits and botánicals |

### EXAMPLE 9

Among many other options, the composition of the oral chewable form of Example 1 comprising active ingredients from Table 9.

**Table 9**

| | | |
|---|---|---|
| CHEWABLE AGAR MATRIX | Vitamin A | Beta-carotene |
| | Vitamin C | Ascórbic ácid |
| | Vitamin D2 | Ergocalciferol |
| | Vitamin E | d-α-Tocopheryl acetate |
| | Vitamin K2 | Menaquinone |
| | Vitamin B1 | Thiamine Mononitrate |
| | Vitamin B3 | Niacinamide |
| | Vitamin B6 | Pyridoxine HCI |
| | Vitamin B9 | Calcium Folate |
| | Vitamin B12 | Cyanocobalamin |
| | Biotin | Biotin |
| | Vitamin B5 | Pantothenic Acid |
| | Choline | Choline Tartrate |
| | Iodine | Potassium Iodide |
| | Magnesium | Magnesium Oxice |
| | Selenium | L- selenomethionine |
| | Zinc | Zinc Citrate |
| | Probiótic | *Bacillus coagulans* |
| | Acerola | Organic Acerola |
| | Fruits and botanicals | Mixture of fruits and botanicals |

### EXAMPLE 10

This example discloses a chewable oral composition of agar gums including the composition of the form of Example 1 and comprising active ingredients from Table 10.

**Table 10**

| | | |
|---|---|---|
| CHEWABLE AGAR MATRIX | Vitamin B6 | Pyridoxine HCI |
| | Vitamin B12 | Cyanocobalamin |
| | Ginseng | *Extract of ginseng* |

### EXAMPLE 11

Another example of a composition in oral chewable form from Example 1 and comprising active ingredients from Table 11.

**Table 11**

| | | |
|---|---|---|
| CHEWABLE AGAR MATRIX | Magnesium | Magnesium Citrate |
| | Melatonin | Melatonin |
| | Extract of passión flower | *Passiflora incarnata L.* |

### EXAMPLE 12

An example of a chewable oral composition from Example 1 comprising the active ingredients of Table 12.

**Table 12**

| | | |
|---|---|---|
| CHEWABLE AGAR MATRIX | Lemon balm extract | *Melissa officinalis* |
| | L- theanine | L- theanine |

### EXAMPLE 13

An example of a chewable oral composition from Example 1, comprising the active ingredients from Table 13.

**Table 13.**

| | | |
|---|---|---|
| CHEWABLE AGAR MATRIX | Saffron extract | *Iridaceae Crocus sativus L.* |
| | Vitamin D3 | Cholecalciferol |

### EXAMPLE 14

Another example of a chewable oral composition from Example 1 comprising the active ingredients of Table 14.

**Table 14**

| | | |
|---|---|---|
| CHEWABLE AGAR MATRIX | Vitamin C | Ascórbic ácid |
| | Zinc | Zinc Citrate |
| | Eldelberry Extract B | *Sambucus nigra* |

### EXAMPLE 15

A final example of a chewable oral composition from Example 1 comprising the active ingredients from Table 15.

**Table 15**

| | | |
|---|---|---|
| CHEWABLE AGAR MATRIX | Magnesium | Magnesium Citrate |

This example 15, in particular a range of 0.1% and 4.0% of agar-agar gelling agent in combination with locust bean gum; sweetener in a range of between 6% to 30%; glycerin in a range of between 1% and 3%; liquid inulin in a range of between 7% and 12%; inulin powder in a range of between 5% and 10%; flavoring in a range of between 0.1% and 1%; citric acid in a range of 0.1% and 0.5%; wax-based coating in a range of between 0.5% and 3%; black carrot in a range of 0.1% and 1%; and, water until completing the composition, in combination with magnesium citrate.

This example 15 defines a composition that particularly comprises magnesium.

Magnesium is a mineral necessary for more than 300 biochemical functions in the human body and there is currently a deficiency of the mineral in the daily diet.

For the present invention, 20% of magnesium had to be exceeded, which normally in state-of-the-art gums generates a high instability of the matrix due to volume and due to the low solubility of magnesium citrate, which according to the present invention was achieved. constituting an agar matrix chewable oral form that effectively comprises between 28% and 32% magnesium citrate.

The chewable oral form of agar matrix according to the present invention comprises a formulation that achieves a balance in the mixture of the aforementioned components to provide a matrix that can be industrially processed and that has stability during the shelf life of 2 years.

The present invention is thus applicable to the nutritional or health area since it provides an oral form with outstanding texture, visual shape and flavor capable of supplying the desired amount of an active ingredient.

Only some preferred embodiments of the invention have been illustrated by way of example.

## Claims

1. Chewable oral form **characterized in that** it comprises a composition of: agar-agar gelling agent in combination with locust bean gum in a range of 0.1% and 4.0% of the total weight of the composition; sweetener in a range of between 6% to 30% of the total weight of the composition; glycerin in a range of between 1% and 3% of the total weight of the composition; liquid inulin in a range of between 7% and 12% of the total weight of the composition; powdered inulin in a range of between 5% and 10% of the total weight of the composition; flavoring in a range of between 0.1% and 1% of the total weight of the composition; citric acid in a range of 0.1% and 0.5% of the total weight of the composition; wax-based coating in a range of between 0.5% and 3% of the total weight of the composition; black carrot in a range of 0.1% and 1% of the total weight of the composition; in combination with active ingredients in a range of between 10% and 60% of the total weight of the composition; and water to complete the composition, wherein
the active ingredient is selected from vitamin D2, vitamin A, vitamin C, vitamin D and its derivatives and / or analogues, vitamin E, B complex vitamins, such as vitamins B1, B3, B5, B9 and B12 and vitamin K2; probiotics; minerals selected from zinc, calcium, magnesium, iron, selenium, chromium, phosphorus, potassium, copper; omega 3 fatty acids selected from EPA and DHA; folic acid, iodine, biotin, choline, lutein or their combinations.

2. Chewable oral form according to claim 1, **characterized in that** the active ingredient is in a range of between 25% and 35% of the total weight of the composition.

3. Chewable oral form according to claim 1 or 2, **characterized in that** the flavoring is selected from strawberry, watermelon, mango, lemon, peppermint, orange, passion fruit, banana, coconut, acerola, blueberry, currant, mandarin, papaya, grapefruit, acai, chamomile and ginger.

4. Chewable oral form according to claim 1 or 2, **characterized in that** the sweeteners comprise sugars based on organic sugar, cane sugar, tapioca syrup and inulin.

5. Process of shaping the chewable oral form of claim 1 or 2, **characterized in that** it comprises:
(i) a first stage of hydration of the agar-agar and locust bean gum gelling agent, which includes mixing agar-agar in combination with locust bean gum between 0.1% and 4.0%, particularly between 0.2% and 2.0% and more particularly between 0.3% and 1.8% of the total weight of the composition, with an amount of sweeteners in a range of between 6% and 10% until a homogeneous mixture is obtained, and then add a quantity of water and stir until complete dissolution of the mixture;
(ii) a second stage of incorporation of components of the agar matrix, where to the mixture of the first stage (i), glycerin is added in an amount between 1% and 3% of the total composition in combination with inulin liquid in an amount of between 7% and 12% of the total composition and inulin powder in an amount of between 5% and 10% of the total composition and optionally mix with an amount of sweetener between 20% and 28% of the total of the composition, and then stir until complete dissolution;
(iii) a third stage of incorporation of an active ingredient, where an amount of the active ingredient and optionally an amount of sweetener of between 7% and 10% of the total composition are previously mixed until a homogeneous mixture is obtained and subsequently add to the mixture of the second stage (ii) and stir until its complete incorporation;
(iv) a fourth stage of cooking the mixture obtained in the third stage (iii) at a temperature between 80° C and 100° C for 30 ± 5 minutes, where the solids must comprise between 72° and 74° Brix; and,
(v) a fifth stage of flavoring that includes transferring the cooked pasta from the fourth stage (iv) to a flavoring tank and adding black carrot in an amount of between 0.1% and 1%, particularly between 0.2% and 0.4% in combination with flavoring in an amount between 0.1% and 1%, particularly between 0.4% to 0.8% and citric acid in an amount between 0.23% and 0.29%.

6. Process according to claim 5 of shaping the chewable oral form of claim 1 , **characterized in that** the process additionally comprises
(vi) a sixth stage of molding and gelling where the mixture from the fifth stage (v) is deposited in molds at a temperature between 75° and 89° C and its temperature for gelling is reduced to a temperature of between 2° C at 8° C; and,
(vii) a seventh stage of demoulding, degreasing and drying, which includes demoulding followed by degreasing, and then drying by spreading the chewable oral forms on stools or drying baskets at 30 ± 3 ° C temperature and 25 ± 5 % relative humidity.

7. Process according to claim 5 of shaping the chewable oral form of claim 1 , **characterized in that** the process additionally comprises:
(viii) an eighth stage of sweetening and packaging, where the dry chewable oral forms are introduced to the sweetening equipment and the sweetener is added until the coating is complete and for this the steam is adjusted between 275 Pa to 413 Pa, with a speed of 1 at 5 Kg unit / min and an amount of sweetener between 6% and 10%, where once sweetened, they are inspected and packed.

8. Process according to claim 6 of shaping the chewable oral form of claim 1, **characterized in that** the drying of the seventh stage (vii) is under conditions of relative humidity 25 ± 5%, with a temperature between 30 ± 3 ° C and laminar flow air velocity of 0.15 m³/sec.

## Patentansprüche

1. Kaubare orale Darreichungsform, **dadurch gekennzeichnet, dass** sie eine Zusammensetzung aus Folgendem umfasst: Agar-Agar-Geliermittel in Kombination mit Johannisbrotkernmehl in einem Bereich von 0,1% und 4,0% des Gesamtgewichts der Zusammensetzung; Süßungsmittel in einem Bereich zwischen 6% bis 30% des Gesamtgewichts der Zusammensetzung; Glycerin in einem Bereich zwischen 1% und 3% des Gesamtgewichts der Zusammensetzung; flüssiges Inulin in einem Bereich zwischen 7% und 12% des Gesamtgewichts der Zusammensetzung; pulverförmiges Inulin in einem Bereich zwischen 5% und 10% des Gesamtgewichts der Zusammensetzung; Aromastoff in einem Bereich zwischen 0,1% und 1% des Gesamtgewichts der Zusammensetzung; Zitronensäure in einem Bereich von 0,1% und 0,5% des Gesamtgewichts der Zusammensetzung; Beschichtung auf Wachsbasis in einem Bereich zwischen 0,5% und 3% des Gesamtgewichts der Zusammensetzung; schwarze Karotte in einem Bereich von 0,1% und 1% des Gesamtgewichts der Zusammensetzung; in Kombination mit Wirkstoffen in einem Bereich zwischen 10% und 60% des Gesamtgewichts der Zusammensetzung; und Wasser zur Vervollständigung der Zusammensetzung, wobei
der Wirkstoff aus Vitamin D2, Vitamin A, Vitamin C, Vitamin D und seinen Derivaten und/oder Analoga, Vitamin E, Vitamin B-Komplexen, wie beispielsweise Vitamin B1, B3, B5, B9 und B12 und Vitamin K2; Probiotika; Mineralstoffen, ausgewählt aus Zink, Calcium, Magnesium, Eisen, Selen, Chrom, Phosphor, Kalium, Kupfer; Omega-3-Fettsäuren, ausgewählt aus EPA und DHA; Folsäure, Jod, Biotin, Cholin, Lutein oder deren Kombinationen ausgewählt ist.

2. Kaubare orale Darreichungsform nach Anspruch 1, **dadurch gekennzeichnet, dass** der Wirkstoff in einem Bereich zwischen 25% und 35% des Gesamtgewichts der Zusammensetzung liegt.

3. Kaubare orale Darreichungsform nach Anspruch 1 oder 2, **dadurch gekennzeichnet, dass** der Aromastoff aus Erdbeere, Wassermelone, Mango, Zitrone, Pfefferminze, Orange, Passionsfrucht, Banane, Kokosnuss, Acerola, Blaubeere, Johannisbeere, Mandarine, Papaya, Grapefruit, Acai, Kamille und Ingwer ausgewählt ist.

4. Kaubare orale Darreichungsform nach Anspruch 1 oder 2, **dadurch gekennzeichnet, dass** die Süßungsmittel Zucker auf Basis von Bio-Zucker, Rohrzucker, Tapiokasirup und Inulin umfassen.

5. Verfahren zur Formgebung der kaubaren oralen Darreichungsform nach Anspruch 1 oder 2, **dadurch gekennzeichnet, dass** es Folgendes umfasst:
(i) eine erste Stufe der Hydratation des Agar-Agar- und Johannisbrotkernmehl-Geliermittels, die das Mischen des Agar-Agars in Kombination mit dem Johannisbrotkernmehl zwischen 0,1% und 4,0%, insbesondere zwischen 0,2% und 2,0% und ganz besonders zwischen 0,3% und 1,8% des Gesamtgewichts der Zusammensetzung mit einer Menge von Süßungsmitteln im Bereich zwischen 6% und 10% umfasst, bis eine homogene Mischung erhalten wird, und dann Hinzugeben einer Menge von Wasser und Rühren, bis sich die Mischung vollständig aufgelöst hat;
(ii) eine zweite Stufe der Einarbeitung von Komponenten der Agarmatrix, wobei der Mischung der ersten Stufe (i) Glycerin in einer Menge zwischen 1% und 3% der Gesamtzusammensetzung in Kombination mit flüssigem Inulin in einer Menge zwischen 7% und 12% der Gesamtzusammensetzung und Inulinpulver in einer Menge zwischen 5% und 10% der Gesamtzusammensetzung zugegeben wird und gegebenenfalls Mischen mit einer Menge von Süßungsmitteln zwischen 20% und 28% der Gesamtzusammensetzung und dann Rühren bis zum vollständigen Auflösen;
(iii) eine dritte Stufe der Einarbeitung eines Wirkstoffs, wobei eine Menge des Wirkstoffs und gegebenenfalls eine Menge des Süßungsmittels von zwischen 7% und 10% der Gesamtzusammensetzung zuvor vermischt wird, bis eine homogene Mischung erhalten wird und anschließend Hinzufügen zur Mischung der zweiten Stufe (ii) und Rühren bis er vollständig eingearbeitet ist;
(iv) eine vierte Stufe des Kochens der Mischung, die im dritten Schritt (iii) erhalten worden ist, bei einer Temperatur zwischen 80°C und 100°C für 30 ± 5 Minuten, wobei die Feststoffe einen Brix-Wert zwischen 72° und 74° aufweisen müssen; und
(v) eine fünfte Stufe der Aromatisierung, die das Überführen des gekochten Teigs aus der vierten Stufe (iv) in einen Aromabehälter und das Hinzufügen von schwarzer Karotte in einer Menge zwischen 0,1% und 1%, insbesondere zwischen 0,2% und 0,4% in Kombination mit Aromastoff in einer Menge zwischen 0,1% und 1%, insbesondere zwischen 0,4% bis 0,8% und Zitronensäure in einer Menge zwischen 0,23% und 0,29%, umfasst.

6. Verfahren nach Anspruch 5 zur Formgebung der kaubaren oralen Darreichungsform nach Anspruch 1, **dadurch gekennzeichnet, dass** es zusätzlich Folgendes umfasst:
(vi) eine sechste Stufe der Formung und Gelierung, in der die Mischung aus der fünften Stufe (v) bei einer Temperatur zwischen 75° und 89°C in Formen gefüllt und ihre Temperatur zum Gelieren auf eine Temperatur zwischen 2°C und 8°C reduziert wird; und
(vii) eine siebte Stufe der Entformung, Entfettung und Trocknung, die das Entformen, gefolgt vom Entfetten und anschließendem Trocknen durch Ausbreiten der kaubaren oralen Darreichungsformen auf Hockern oder Trockenkörben bei einer Temperatur von 30 ± 3°C und einer relativen Luftfeuchtigkeit von 25 ± 5% umfasst.

7. Verfahren nach Anspruch 5 zur Formgebung der kaubaren oralen Darreichungsform nach Anspruch 1, **dadurch gekennzeichnet, dass** es zusätzlich Folgendes umfasst:
(viii) eine achte Stufe des Süßens und Verpackens, bei dem die trockenen kaubaren oralen Darreichungsformen in die Süßungsvorrichtung eingeführt werden und das Süßungsmittel hinzugegeben wird, bis die Beschichtung vollständig ist und hierfür wird der Dampfdruck zwischen 275 Pa und 413 Pa eingestellt, mit einer Geschwindigkeit von 1 bei 5 kg Einheiten/min und einer Menge an Süßungsmitteln zwischen 6% und 10%, wobei sie, wenn sie einmal gesüßt worden sind, geprüft und verpackt werden.

8. Verfahren nach Anspruch 6 zur Formgebung der kaubaren oralen Darreichungsform nach Anspruch 1, **dadurch gekennzeichnet, dass** das Trocknen der siebten Stufe (vii) bei einer relativen Luftfeuchtigkeit von 25 ± 5%, bei einer Temperatur zwischen 30 ± 3°C und einer laminaren Luftströmungsgeschwindigkeit von 0,15 m³/sec durchgeführt wird.

## Revendications

1. Forme orale masticable **caractérisée en ce qu'**elle comprend une composition comprenant :
de l'agent gélifiant d'agar-agar en combinaison avec de la gomme de caroube en une quantité comprise entre 0,1 % 4,0 % du poids total de la composition,
de l'édulcorant en une quantité comprise entre 6 % et 30 % du poids total de la composition,
du glycérol en une quantité comprise entre 1 % et 3 % du poids total de la composition,
de l'inuline liquide en une quantité comprise entre 7 % et 12 % du poids total de la composition,
de l'inuline en poudre en une quantité comprise entre 5 % à 10 % du poids total de la composition,
de l'agent aromatisant en une quantité comprise entre 0,1 % à 1 % du poids total de la composition,
de l'acide citrique en une quantité comprise entre 0,1 % et 0,5 % du poids total de la composition,
de l'enrobage à base de cire en une quantité comprise entre 0,5 % et 3 % du poids total de la composition,
de la carotte noire en une quantité comprise entre 0,1 % à 1 % du poids total de la composition,
en combinaison avec des substances actives en une quantité comprise entre 10 % à 60 % du poids total de la composition, et de l'eau jusqu'à compléter la composition,
dans laquelle
la substance active est sélectionnée parmi de la vitamine D2, de la vitamine A, de la vitamine C, de la vitamine D et ses dérivés et/ou analogues, de la vitamine E, des vitamines du complexe B, telles que des vitamines B1, B3, B5, B9 et B12, et de la vitamine K2, des probiotiques, des minéraux sélectionnés parmi du zinc, du calcium, du magnésium, du fer,
du sélénium, du chrome, du phosphore, du potassium, du cuivre, des acides gras oméga 3 sélectionnés parmi de l'EPA et du DHA , de l'acide folique, de l'iode, de la biotine, de la choline, de la lutéine ou leurs combinaisons.

2. Forme orale masticable selon la revendication 1, **caractérisée en ce que** la substance active est présente en une quantité comprise entre 25 % et 35 % du poids total de la composition.

3. Forme orale masticable selon la revendication 1 ou 2, **caractérisée en ce que** l'agent aromatisant est sélectionné parmi la fraise, la pastèque, la mangue, le citron, la menthe poivrée, l'orange, les fruits de la passion, la banane, la noix de coco, l'acérola, la myrtille, le cassis, la mandarine, la papaye, le raisin, l'açaï, la camomille et le gingembre.

4. Forme orale masticable selon la revendication 1 ou 2, **caractérisée en ce que** les édulcorants comprennent des sucres à base de sucre biologique, de sucre de canne, de sirop de tapioca et d'inuline.

5. Procédé de formation de la forme orale masticable selon la revendication 1 ou 2, **caractérisé en ce qu'**il comprend :
i) une première étape d'hydratation de l'agent gélifiant d'agar-agar et de gomme de caroube, qui inclut le mélange de l'agar-agar combiné à de la gomme de caroube en une quantité comprise entre 0,1 % 4,0 %, en particulier entre 0,2 % et 2,0 % et plus particulièrement entre 0,3 % et 1,8 % du poids total de la composition, avec une quantité d'édulcorants comprise entre 6 % et 10 % jusqu'à l'obtention d'un mélange homogène, puis l'ajout d'une quantité d'eau et une agitation jusqu'à dissolution complète du mélange,
ii) une deuxième étape d'incorporation des composants dans la matrice d'agar-agar, dans laquelle du glycérol est ajouté au mélange de la première étape i) en une quantité comprise entre 1 % et 3 % du poids total de la composition, en combinaison avec de l'inuline liquide en une quantité comprise entre 7 % et 12 % de la composition totale et de la poudre d'inuline en une quantité comprise entre 5 % et 10 % de la composition totale et incluant optionnellement un mélange avec une quantité d'édulcorant comprise entre 20 % et 28 % du total de la composition, puis une agitation jusqu'à dissolution complète,
iii) une troisième étape d'incorporation d'une substance active, dans laquelle une quantité de la substance active et optionnellement une quantité d'édulcorant comprise entre 7 % et 10 % de la composition totale sont préalablement mélangées jusqu'à l'obtention d'un mélange homogène et ajoutées ensuite au mélange de la deuxième étape (ii), avec une agitation jusqu'à une incorporation complète,
iv) une quatrième étape de cuisson du mélange obtenu dans la troisième étape (iii) à une température comprise entre 80 °C et 100 °C pendant 30 ± 5 minutes, où les solides doivent présenter un taux de matières sèches solubles de 72 à 74 °Brix, et
v) une cinquième étape d'aromatisation qui inclut le transfert de la pâte cuite provenant de la quatrième étape (iv) jusque dans une cuve d'aromatisation et l'ajout de carotte noire en une quantité comprise entre 0,1 % et 1 %, en particulier entre 0,2 % et 0,4 % en combinaison avec de l'agent aromatisant en une quantité comprise entre 0,1 % et 1 %, particulièrement entre 0,4 % et 0,8 % et de l'acide citrique en une quantité comprise entre 0,23 % et 0,29 %.

6. Procédé selon la revendication 5 de formation de la forme orale masticable selon la revendication 1, **caractérisé en ce que** le procédé comprend en outre :
vi) une sixième étape de moulage et de gélification dans laquelle le mélange provenant de la cinquième étape (v) est déposé dans des moules à une température entre 75° et 89 °C et sa température est abaissée à une température comprise entre 2 °C et 8 °C pour la gélification, et
vii) une septième étape de démoulage, de dégraissage et de séchage, qui inclut un démoulage suivi d'un dégraissage, puis un séchage en répartissant les formes orales masticables sur des plateaux ou des bacs de séchage à une température de 30 ± 3 °C et à une humidité relative de 25 ± 5 %.

7. Procédé selon la revendication 5 de formation de la forme orale masticable selon la revendication 1, **caractérisé en ce que** le procédé comprend en outre :
viii) une huitième étape d'édulcoration et de conditionnement, dans laquelle les formes orales masticables sèches sont introduites dans l'appareillage d'édulcoration et l'édulcorant est ajouté jusqu'à ce que l'enrobage soit complet et, pour cela, la vapeur est réglée entre 275 Pa et 413 Pa, avec un débit de 1 à 5 kg / min, et une quantité d'édulcorant comprise entre 6 % et 10 %, puis, une fois édulcorées, elles sont examinées et conditionnées.

8. Procédé selon la revendication 6 de formation de la forme orale masticable selon la revendication 1, **caractérisé en ce que** le séchage de la septième étape (vii) est réalisé dans des conditions d'humidité relative de 25 ± 5 %, de température de 30 ± 3 °C et de débit d'air sous un écoulement laminaire de 0,15 m³/sec.
